# EUROPEAN PATENT SPECIFICATION

(11) **EP 0 519 976 B1**
(45) Date of publication and mention of the grant of the patent: **26.02.1997**
(21) Application number: 91905955.0
(22) Date of filing: 12.03.1991
(51) Int. Cl.: C07K 7/06, C07K 7/08, C07K 14/525, A61K 38/04

(54) **NEUTROPHIL STIMULATING PEPTIDES**
NEUTROPHILE STIMULIERENDE PEPTIDE
PEPTIDES STIMULANT LES POLYNUCLEAIRES NEUTROPHILES

(30) Priority: 12.03.1990 AU 9065/90
(43) Date of publication of application: 30.12.1992
(73) Proprietor: PEPTIDE TECHNOLOGY LTD, Dee Why, NSW 2099 (AU)
(72) Inventor: RATHJEN, Deborah, Anne, Thornleigh, NSW 2120 (AU); FERRANTE, Antonio, Mount Osmond, S.A. 5064 (AU)
(74) Representative: Sheard, Andrew Gregory
(86) International application number: AU9100086
(87) International publication number: WO9113908

(56) References cited:
- WO-A-91/02078
- WO-A-91/14702
- WO-A-91/17180
- AU-B- 4 465 285
- Proc. Natl. Acad. Sci USA, Vol. 84, pp. 8829-8833, Dec. 1987, S.H. SOCHER et al.

## Description

The present invention relates to peptides having neutrophil stimulating activity, and to use of these peptides as therapeutic agents.

Tumour necrosis factor (TNF) was first identified as a factor found in the serum of Bacillus Calmette-Guerin treated mice which caused haemorraghic regression of certain transplanted tumours and had cytolytic activity on several transformed cell lines in vitro (Carswell et al, PNAS 72, 3666 - 3670; Helson et al, 1975, Nature 258, 731-732). TNF, a product of activated macrophages, has subsequently been shown to be a primary mediator in the pathology of endotoxic shock (Tracey et al 1986, Science 234, 470-474). In addition to its pathological effects TNF also has a central role in host defenses against viral, bacterial and parasitic pathogens.

The cellular targets of TNF important in host defence include neutrophils, eosinophils, monocyte/macrophages and lymphocytes. Within this context TNF is a major mediator of neutrophil activation. TNF stimulates enhanced phagocytosis (Shalaby et al 1985, J.Immunol., 135, 2069-2073), enhanced production of superoxide anions (Teujiimoto et al, 1986, Biochem. Biophys. Res. Commun., 137, 1094-1100), release of lysozyme and hydrogen peroxide and causes neutrophil degranulation (Klebanoff et al, 1986, J.Immunol., 136, 4220-4225). Neutrophils also show enhanced microbicidal and tumouricidal activity when stimulated by TNF (Shalaby et al, 1985, J.Immunol., 135, 2069-2073; Djeu et al, 1986, J.Immunol., 137, 2980-2984; Blanchard et al, 1989, J.Leuk. Biol., 45, 538-545). It has been hypothesized that the cytostatic effect of TNF is mediated by high local concentrations of hydrogen peroxide produced by neutrophils (Shau 1986, J.Immunol., 141, 234-240).

TNF pretreatment enhances the response of neutrophils to N-formyl-L-methionyl-L-leucyl-L-phenylalanine (F-met-leu-phe) and phorbol myristate acetate through specific receptors (Ferrante et al 1988, Int. Arch. Allergy Appl. Immunol., 86, 82-91). Neutrophils accumulate at sites of inflammation, caused in part by the increased expression of complement receptors by TNF (Berger et al 1988, Blood 71, 151-158). Further TNF causes neutrophil emigration into skin (Cybuisky et al 1988, J. Immunol. 140, 3144-3149).

Neutrophil function is known to be depressed in a number of viral, bacterial and parasitic infections (Abramson and Mills, 1988, Rev. Infect. Dis., 10, 326-341; Ferrante et al, 1989, Immunol. Letts., 22, 301-6). Depressed neutrophil function has, for example, been described in Acquired Immune Deficiency Syndrome (Thorsen et al, 1989, AIDS, 3, 651-653; Ellis et al, 1988, J. Infect. Dis., 158, 1268-1276; Murphy et al, 1988, J. Infect. Dis., 158, 627-630). Clearly TNF, which appears to play an important role in neutrophil activation both in vitro and in vivo as described above, given exogenously has the potential to overcome these neutrophil defects. The administration of TNF or indeed overproduction of TNF is, however, associated with severe side effects and the manifestation of pathology such as thrombocytopaenia, lymphocytopaenia, hepatotoxicity, renal impairment and hypertension.

Socher *et al* (PNAS, **84**: 8829-8853) disclose a peptide having an amino acid sequence corresponding to the 65-79 fragment of TNF.

The present inventors have identified novel peptides derived from the primary amino acid sequence of human TNF which stimulate neutrophil activity. These peptides have indicated that the region of amino acids 54 to 94 of human TNF has previously undiscovered neutrophil stimulating activity. This observation has important clinical applications as treatment with such peptides would be expected to restore depressed or aberrant neutrophil activity, but would not be expected to cause the severe side effects associated with the therapeutic use of the whole TNF molecule.

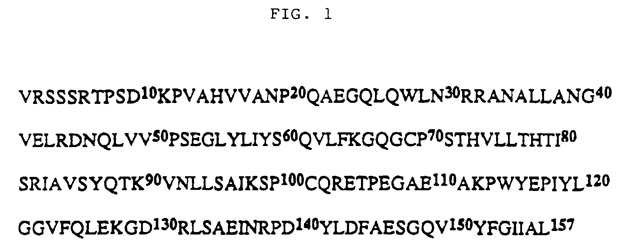

Accordingly, in a first aspect the present invention consists in a peptide having an amino acid sequence substantially corresponding to amino acids 54 to 94 of Figure 1 or a part thereof, the peptide being characterized in that the peptide is capable of eliciting superoxide production by neutrophils and of priming neutrophils for an enhanced respiratory burst following treatment with N-formyl-L-methionyl-L-leucyl-L-phenylalanine.

In a preferred embodiment of the present invention the peptide has an amino acid sequence corresponding to amino acids 54 to 94 of Figure 1.

In a preferred embodiment of the present invention the peptide has an amino acid sequence corresponding to amino acids 63 to 83 of Figure 1.

In another preferred embodiment of the present invention the peptide has an amino acid sequence corresponding to amino acids 54 to 68 of Figure 1.

In yet another preferred embodiment of the present invention the peptide has an amino acid sequence corresponding to amino acids 73 to 94 of Figure 1.

In yet a further preferred embodiment of the present invention, the peptide has amino acid sequence corresponding to amino acids 70 to 80 of Figure 1.

As will be appreciated by those skilled in the art from the description which follows the present inventors have demonstrated that the region of human TNF from amino acid 54 to amino acid 94 plays an important functional role in the stimulation of neutrophils. Further, the present inventors have produced 4 peptides namely peptides 304, 308, 309 and 395 (as referred to herein) which have neutrophil stimulating activity.

In a further aspect, the present invention consists in a method of treating a subject having depressed neutrophil function, the method comprising administering to the subject a therapeutic amount of the peptide of the first aspect of the present invention.

In a preferred embodiment of this aspect of the present invention the subject is suffering from acquired immune deficiency syndrome.

Peptide 308, through slective effects on neutrophil degranulation may be administered to individuals suffering from inflammatory syndromes e.g. rheumatoid arthritis, adult respiratory distress syndrome.

In order that the nature of the present invention may be more clearly understood, preferred forms thereof will now be described with reference to the following examples, and Figures in which:-
Figure 1 shows the amino acid sequence of human TNF;
Figure 2 shows the effects of peptides 304 (○), 308 (□) and 309 (Δ) on the fMLP induced human neutrophil response. Peptides were used at 100µg/10⁶ in the 20 min pre-incubation step,
Figure 3 shows the kinetics of the chemiluminescence response elicited by Peptide 395 ( 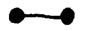 ; 395 (50µg) + fMLP; 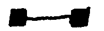 ; 395 (50µg); 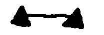 HBSS + fMLP; 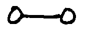 HBSS); and

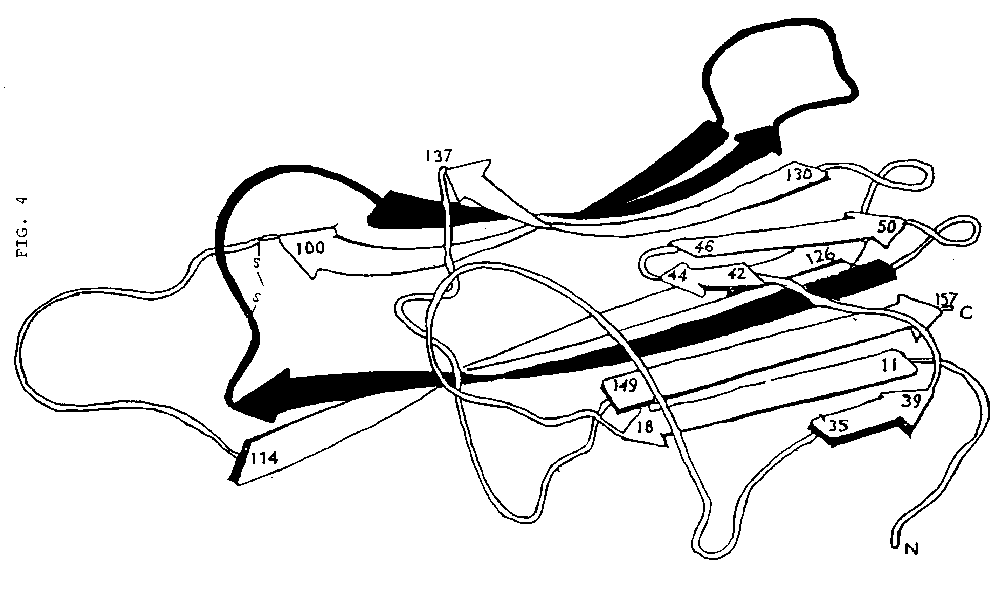

Figure 4 is a representation of the TNFα monomer showing the position of the neutrophil stimulating peptides.

### Production of human TNF peptides tested for neutrophils stimulatory activity.

The following peptides were synthesised and are described using the I.U.P.A.C. one-letter code abbreviations for amino acid residues with the TNF sequence region indicated in brackets.
peptide 275
   A K P W Y E P I Y L (111-120)
peptide 301
   V R S S S R T P S D K P V A H V V A (1-18)
peptide 302
   L R D N Q L V V P S E G L Y L I (43-58)
peptide 303
   L S A I K S P C Q R E T P E G A (94-109)
peptide 304
   L F K G Q G C P S T H V L L T H T I S R I (63-83)
peptide 305
   L S A E I N R P D Y L D F A E S G Q V (132-150)
peptide 306
   V A H V V A N P Q A E G Q L (13-26)
peptide 307
   A E G Q L Q W L N R R A N A L L A N G (22-40)
peptide 308
   G L Y L I Y S Q V L F K G Q G (54-68)
peptide 309
   H V L L T H T I S R I A V S Y Q T K V N L L (73-94)
peptide 323
   T I S R I A V S Y Q T (79-89)

These peptides were synthesised using the following general protocol.

All peptides were synthesised using the Fmoc-polyamide method of solid phase peptide synthesis (Atherton et al, 1978, J. Chem. Soc. Chem. Commun., 13, 537-539). The solid resin used was PepSyn KA which is a polydimethyacrylamide gel on kieselguhr support with 4-hydroxymethylphenoxyacetic acid as the functionalised linker (Atherton et al, 1975, J. Am. Chem. Soc., 97, 6584-6585).

The carboxy terminal amino acid is attached to the solid support by a DCC/DMAP-mediated symmetrical-anhydride esterification.

All Fmoc-groups are removed by piperidine/DMF wash and peptide bonds are formed either via pentafluorophenyl active esters or directly by BOP/NMM/HOBt (Castro's reagent) except for certain amino acids as specified in Table 1.

Side chain protection chosen for the amino acids are removed concomitantly during cleavage with the exception of Acm on cysteine which is left on after synthesis

**TABLE 1**

| Amino acid | Protecting group | Coupling Method |
|---|---|---|
| Arg | Mtr or Pmc | Either |
| Asp | OBut | Either |
| Cys | Acm (permanent) | Either |
| Glu | OBut | Either |
| His | Boc | OPfp only |
| Lys | Boc | Either |
| Ser | But | BOP only |
| Thr | But | BOP only |
| Tyr | But | Either |
| Asn | none | OPfp only |
| Gln | none | OPfp only |

### Cleavage and Purification

Peptide 302. Peptide is cleaved from the resin with 95% TFA and 5% thioanisole (1.5 h) and purified on reverse phase C4 column. (Buffer A - 0.1% aqueous TFA, Buffer B - 80% ACN 20% A)

Peptide 304. Peptide is cleaved from the resin with 95% TFA and 5% phenol (5 h) and purified on reverse phase C4 column. (Buffer A - 0.1% aqueous TFA, Buffer B - 80% ACN 20% A).

Peptide 308. Peptide is cleaved from the resin with 95% TFA and 5% water (1.5 h) and purified on reverse phase C4 column. (Buffer A - 0.1% aqueous TFA, Buffer B - 80% ACN 20% A).

Peptide 309. Peptide is cleaved from the resin with 95% TFA and 5% thioanisole and purified on reverse phase C4 column. (Buffer A - 0.1% aqueous TFA, Buffer B - 80% ACN 20% A).

In addition, the following synthetic fragments of peptide 309 were synthesized. These peptides had the following amino acid sequence with the TNF sequence region indicated in brackets.
Peptide 393
   L T H T I S R I A (76-84).
Peptide 394
   S R I A V S Y Q T K V N L L (81-94).
Peptide 395
   P S T H V L L T H T I (70-80).
Peptide 396
   A V S Y Q T K V N L L (84-94).

### Effect of TNF peptides on neutrophil function.

### Chemiluminesence assay.

This assay examined the effect of TNF peptides on priming for a neutrophil F-met-leu-phe response as described by Ferrante et al, 1988, (Int. Arch. Allergy Appl. Immunol, 86, 82-91). Purified human neutrophils were pretreated with peptide for 20 minutes before the addition of f-met-leu-phe. The lucigenin dependent chemiluminescence response, which reflects superoxide production, was then measured. The results obtained are set out in Table 2 and are expressed as mV of lucigenin dependent chemiluminescence and represent the maximal cell activity attained.

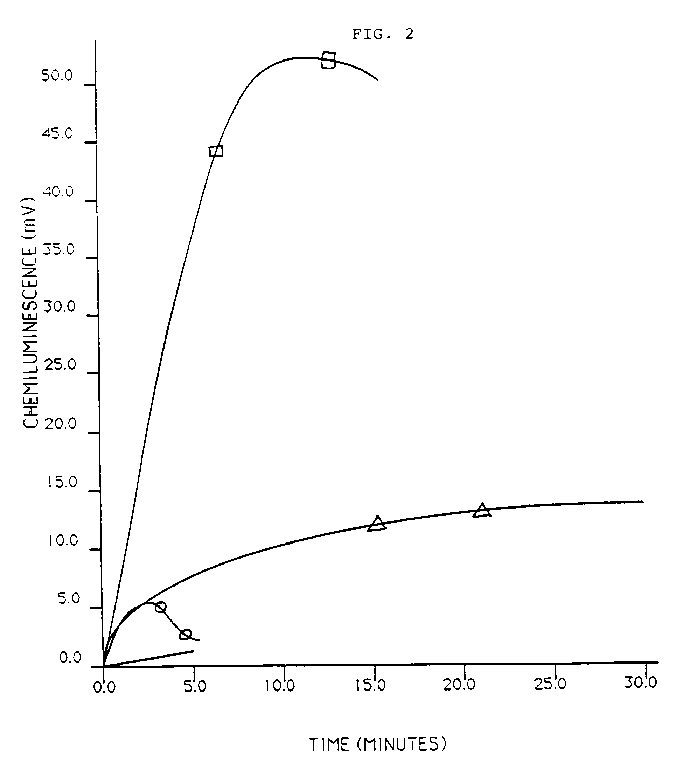

In addition, the effects of peptide 304,308 and 309 are shown graphically in Figure 2.

This experiment was repeated with peptides 304, 308 and 309. The results obtained as shown in Table 3.

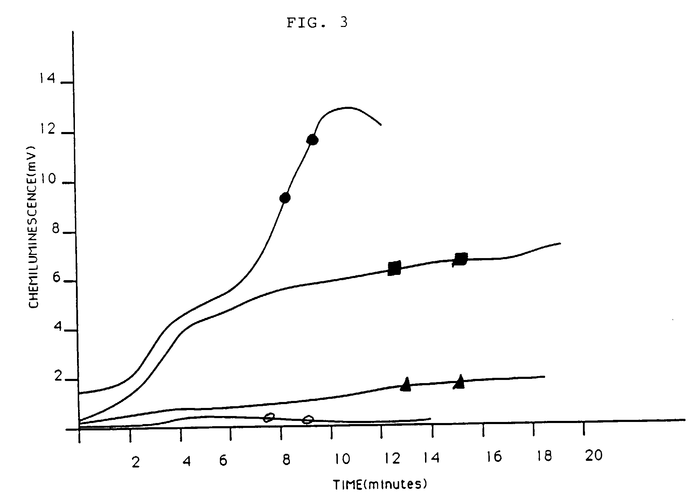

The experiment was also conducted using peptides 393, 394, 395 and 396. Of these peptides only peptide 395 was able to stimulate the neutrophil respiratory burst (Table 4). The effect of peptide 395 was dose dependent as shown by the results of 3 experiments (Table 5). The kinetics of the chemiluminescence response elicited by peptide 395 is shown in Figure 3. Peptide 395 displays improved solubility over peptide 309.

**TABLE 2**

| Peptide | Concentration µg/10⁶ cells) | | | | |
|---|---|---|---|---|---|
| | 0 | 1 | 10 | 100 | 500 |
| 275 | 1.02 | 0.99 | 0.69 | 0.43 | 0.80 |
| 301 | 0.34 | 0.93 | 0.74 | 0.55 | 1.10 |
| 302 | 0.37 | 0.16 | 0.18 | 0.29 | |
| 303 | 0.37 | 0.23 | 0.17 | 0.22 | |
| 304 | 0.37 | 0.18 | 0.43 | 2.56 | 2.76 |
| 305 | 0.37 | 0.27 | 0.36 | 0.24 | |
| 306 | 0.37 | 0.27 | 0.35 | 0.23 | |
| 307 | 0.37 | 0.35 | 0.37 | 0.42 | |
| 323 | 0.37 | 0.23 | 0.17 | 0.47 | |
| 308 | 0.37 | 0.91 | 4.80 | 49.52 | |
| 309 | 0.37 | 0.38 | 0.98 | 13.44 | |

Results are expressed as mV of lucigenin dependent chemiluminescence and respresent peak of response i.e. the maximal cell activity attained.

**TABLE 3**

| Peptide | Peptide concentration (µg/10⁶ cells) | | |
|---|---|---|---|
| | 0 | 10 | 100 |
| 304 | 0.04 | 0.36 | 0.64 |
| 304 + fMLP | 0.71 | 0.91 | 6.97 |
| | | | |
| 308 | 0.04 | 1.00 | 11.76 |
| 308 + fMLP | 0.42 | 2.74 | 28.56 |
| | | | |
| 309 | 0.04 | 0.31 | 0.69 |
| 309 + fMLP | 0.42 | 2.46 | 12.84 |

**Table 4**

| Comparisons of 309 and its subpeptides on neutrophil respiratory burst | |
|---|---|
| Treatment (100 µg peptide) | Chemiluminescence (mV) |
| Diluent | 0.58 |
| 309 | 4.70 |
| 393 | 0.31 |
| 394 | 0.33 |
| 395 | 5.32 |
| 396 | 0.70 |

**Table 5**

| Effect of 395 on neutrophil respiratory burst | | | |
|---|---|---|---|
| Treatment | Chemiluminescence (mV) | | |
| | Exp. 1 | Exp. 2 | Exp. 3 |
| Diluent | 0.58 | 0.68 | 0.38 |
| fMLP | 1.53 | 3.53 | 1.96 |
| 1µg 395 | 3.25 | 0.89 | 0.03 |
| 1µg 395 + fMLP | 3.36 | 4.55 | 0.29 |
| 10µg 395 | 4.92 | 3.97 | 0.64 |
| 10µg 395 +fMLP | 7.31 | 9.10 | 2.34 |
| 50µg 395 | 8.01 | 10.81 | |
| 50µg 395 + fMLP | 12.58 | 22.09 | |
| 100µg 395 | 2.36 | 19.14 | 5.26 |
| 100µg 395 + fMLP | 5.29 | 18.10 | 10.59 |
| 100µg 309 | 5.98 | 6.68 | 1.24 |
| 100µg 309 + fMLP | 27.44 | 22.77 | 6.69 |

### Effect on Superoxide Formation

The effect of peptides 308 and 309 on superoxide formation was examined by the cytochrome reduction assay, according to the procedure of Ferrante, 1989 (Infection and Immunity), 57: 2115-2122). The results, expressed as n moles of O₂/5x10⁵ cells as set out in Table 6.

**TABLE 6**

| Peptide | Peptide concentration (µg/5 x 10⁵ cells) | | |
|---|---|---|---|
| | 0 | 10 | 100 |
| 308 | 0.270 | 2.78 | 4.892 |
| 308 + fMLP | 2.757 | 5.00 | 6.729 |
| | | | |
| 309 | 0.270 | 0.62 | 2.30 |
| 309 + fMLP | 2.757 | 3.87 | 5.14 |

### Effect of TNF peptides on neutrophil random migration

Migration of cells is an important property by which cells reach infection sites. Their accumulation at these sites is also dependent on the capacity of inflammatory mediators to inhibit their migration out of the sites. The present inventors have examined TNF and peptide 304, 308 and 309 for their effect on the migration of neutrophils.

In these experiments neutrophils were pre-treated with the peptide or TNF and then examined their ability to migrate out of wells in agarose as described by Ferrante et al, 1988, (Arch. Allergy Appl. Immunol. 86:82-91). The results are shown in Table 7. The results show that TNF was only partially migration inhibitory at 100 units/10⁶ cells. Both peptides 308 and 309 were potent migration inhibitors, however, peptide 304 was found to be chemokinetic (it stimulated cell migration).

**TABLE 7**

| Treatment | Inhibition of Migration (µg/10⁶ cells) | | |
|---|---|---|---|
| | 0 | 10 | 100 |
| TNF | ND | ND | 4% |
| 304* | -16% | -43% | -883% |
| 308 | 0 | 0 | 100% |
| 309 | 0 | 0 | 100% |

| | | | |
|---|---|---|---|
| *Peptide 304 was found to stimulate (chemokinetic) | | | |

### Chemotactic properties of TNF and peptides

The chemotactic properties of TNF and peptides 304, 308 and 309 were examined using the following method: 3ml of molten 2% agarose was mixed with 3ml of 2x concentrated medium 199 containing foetal calf serum (10%) and poured into Petri dishes. Sets of 3 wells of 2.5mm diameter, each 3mm apart, were cut in the agarose. 5µl of neutrophils (2 x 10⁵ cells) were added to the inner well, with chemotactic agent or control medium added to the outer wells. Migration at various time intervals was then measured. The results of these experiments are shown in Table 8.

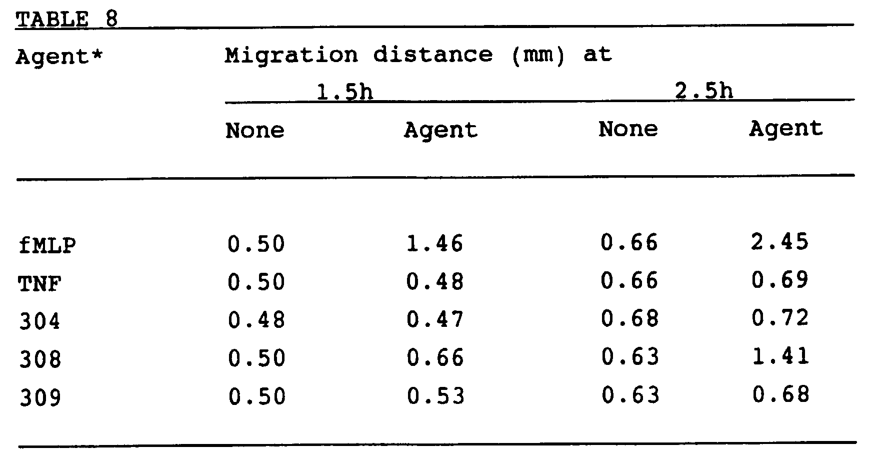

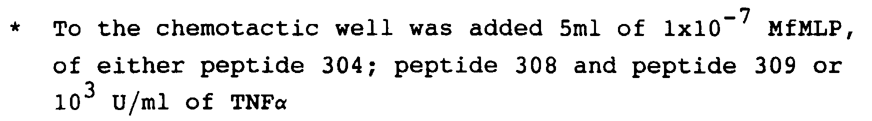

### Effect of TNF Peptides on Neutrophil Degranulation

The conditions of measuring degranulation were as described by Ferrante A, 1989, (Infect and Immunity 57, 3110-3115). In these studies 100µl of neutrophils (10⁷/ml) were incubated for 20min at 37 °C after which 10µl of cytochalasin B was added. After 10 min incubation the volume of cell suspension was made up to 1 ml with Hanks Balanced Salt Solution (HBSS). The cell-free supernatants were collected and analysed for enzyme levels after a further incubation at 37°C. β-Glucuronidase activity was measured fluorimetrically by using 4-methylumbelliferyl-β-D-glucuronide as substrate. This involved incubating 50µl of 2.5 mM substrate in 0.1 M citric acid -sodium phosphate buffer, pH 4.5, at 37°C for 3 h. The reaction was stopped by adding 1.5 ml of 0.2 M glycine-sodium hydroxide buffer, pH 10.7 and the fluorescence of the liberated 4-methylumbelliferone was quantitated by using excitation and emission wavelengths of 336 and 446 nm, respectively. Vitamin B₁₂ binding protein was measured using ⁵⁷Co-vitamin B₁₂. This assay is based on the principle that the binding protein binds to the ⁵⁷Co-Vitamin B₁₂ and as a result the radioactive vitamin B₁₂ does not bind to charcoal. The resultant radioactivity in the supernatant can then be equated to the concentration of vitamin B₁₂-binding protein in the sample. The results of these experiments are set out in Table 9 A and B.

**Table 9**

| Effect of TNF Peptides on Neutrophil Degranulation | | | | |
|---|---|---|---|---|
| Neutrophils were treated with 100µg/10⁶ cells of 304, 305 or 308±fMLP (in the presence of CytoB). | | | | |
| A. | | | | |
| Treatment | β-glucuronidase release (%) | | | |
| | Exp. 1 | Exp. 2 | Exp. 3 | Exp. |
| HBSS | 3.63 | 1.84 | 2.72 | 6.23 |
| HBSS + fMLP | 23.62 | 41.41 | 40.19 | 27.54 |
| | | | | |
| 304 | 3.63 | 3.14 | 2.52 | 9.82 |
| 304 + fMLP | 26.95 | 36.43 | 35.34 | 36.65 |
| | | | | |
| Control peptide | - | - | - | 13.41 |
| Control peptide + fMLP | - | - | - | 35.69 |
| | | | | |
| 308 | 0.8 | 0.65 | 2.76 | 0.72 |
| 308 + fMLP | 17.57 | 28.86 | 17.86 | 18.20 |

| B. | | | | |
|---|---|---|---|---|
| Treatment | Vitamin B₁₂ Binding Protein | | | |
| | Exp. 1 | Exp. 2 | Exp. 3 | Exp. 4 |
| HBSS | 9.21 | 9.27 | 9.67 | 4.80 |
| HBSS + fMLP | 28.85 | 27.91 | 45.31 | 27.33 |
| 304 | 11.40 | 10.82 | 13.06 | 8.42 |
| 304 + fMLP | 43.76 | 35.60 | 59.15 | 37.12 |
| Control peptide | - | - | - | 7.49 |
| Control peptide + fMLP | - | - | - | 38.62 |
| 308 | 2.00 | 2.08 | 5.70 | 2.25 |
| 308 + fMLP | 35.81 | 27.59 | 26.55 | 21.51 |

The effects of TNFα peptides on stimulation of neutrophil respiratory burst, degranulation, migration inhibition, chemokinesis and chemotaxis were investigated. As can be seen from the results set out above only peptides 304, 308 and 309 were found to prime human neutrophils for the respiratory burst associated with f-met-leu-phe treatment, i.e. in a manner analogous to that of TNFα. Together these peptides comprise the primary amino acids sequence region of amino acids 54 to 94 of human TNFα. Peptide 308 is a particularly potent primer of neutrophils in this assay.

It is to be noted, however, that peptide 323 which has a sequence which corresponds to amino acids 79 to 89 of human TNF was not found to be capable of priming neutrophils for the respiratory burst associated with f-met-leu-phe treatment. The reason for the lack of neutrophil stimulating activity of this peptide has not as yet been ellucidated, however, one hypothesis for the lack of activity of this peptide may be that peptide 323 does not include the amino acid residues which bind to the TNF receptor on the neutrophils.

Peptides 308 and 309 have also been found to be potent inhibitors of neutrophil migration whilst peptide 304 has been found to be chemokinetic. Peptide 308 has also been found to be strongly chemotactic.

The effects of TNF peptides 304 and 308 on degranulation of neutrophils (Table 9) showed that peptide 308 decreased the release of the contents of both the specific and the azurophilic granules as measured by the release of Vitamin B₁₂ binding protein and β-glucuronidase release respectively. This effect of peptide 308 was still apparent following stimulation with fMLP. In contrast, peptide 304 had no effect on neutrophil degranulation in the absence of fMLP. In the presence of fMLP peptide 304 enhanced release from specific granules but not azurophilic granules.

## Claims

1. A peptide having an amino acid sequence corresponding to amino acids 54 to 94 of Figure 1, or a part thereof, wherein the peptide is capable of priming neutrophils for a superoxide production and an enhanced respiratory burst following treatment with N-formyl-L-methionyl-L-leucyl-L-phenylalanine.

2. A peptide as claimed in claim 1 in which the peptide has an amino acid sequence corresponding to amino acids 54 to 94 of Figure 1.

3. A peptide as claimed in claim 1 in which the peptide has an amino acid sequence corresponding to amino acids 54 to 68 of Figure 1.

4. A peptide as claimed in claim 1 in which the peptide has an amino acid sequence corresponding to amino acids 73 to 94 of Figure 1.

5. A peptide as claimed in claim 1 in which the peptide has an amino acid sequence corresponding to amino acids 70 to 80 of Figure 1.

6. A compound having a three dimensional structure which corresponds to the three dimensional structure of the peptide as claimed in any one of claims 1 to 5, in which the compound is capable of priming neutrophils for a respiratory burst following treatment with N-formyl-L-methionyl-L-leucyl-L-phenylalanine.

7. A pharmaceutical formulation comprising a peptide as claimed in any one of claims 1 to 5 and any acceptable pharmaceutical carrier or diluent.

8. A peptide as claimed in any one of claims 1 to 5 or a compound as claimed in claim 6 for use in medicine.

9. Use of a peptide as claimed in any one of claims 1 to 5 or a compound as claimed in claim 6 in the preparation of an agent for the treatment or prophylaxis of diseases characterised by depressed neutrophil function.

10. Use as claimed in claim 9 in which the subject is suffering from either acquired immune deficiency syndrome or cancer.

11. Use of a peptide as claimed in claim 3 in the preparation of an agent for the treatment of a subject suffering from an inflammatory syndrome.

12. Use as claimed in claim 11 in which the inflammatory syndrome the subject is suffering from is rheumatoid arthritis or adult respiratory distress syndrome.

## Patentansprüche

1. Peptid mit einer Aminosäuresequenz, die den Aminosäuren 54 bis 94 von Figur 1 entspricht, oder ein Teil davon, wobei das Peptid Neutrophile zur Superoxidproduktion und zu einer erhöhten Stoffwechselaktivierung ("respiratory burst") im Anschluß an eine Behandlung mit N-Formyl-L-methionyl-L-leucyl-L-phenylalanin anregen kann.

2. Peptid nach Anspruch 1, wobei das Peptid eine Aminosäuresequenz aufweist, die den Aminosäuren 54 bis 94 in Figur 1 entspricht.

3. Peptid nach Anspruch 1, wobei das Peptid eine Aminosäuresequenz aufweist, die den Aminosäuren 54 bis 68 in Figur 1 entspricht.

4. Peptid nach Anspruch 1, wobei das Peptid eine Aminosäuresequenz aufweist, die den Aminosäuren 73 bis 94 in Figur 1 entspricht.

5. Peptid nach Anspruch 1, wobei das Peptid eine Aminosäuresequenz aufweist, die den Aminosäuren 70 bis 80 in Figur 1 entspricht.

6. Verbindung mit einer dreidimensionalen Struktur, die der dreidimensionalen Struktur des Peptids gemäß einem der Ansprüche 1 bis 5 entspricht, wobei die Verbindung Neutrophile zu einer Stoffwechselaktivierung ("respiratory burst") im Anschluß an eine Behandlung mit N-Formyl-L-methionyl-L-leucyl-L-phenylalanin anregen kann.

7. Arzneimittel, umfassend ein Peptid nach einem der Ansprüche 1 bis 5 und einen pharmazeutisch verträglichen Träger oder Verdünnungsmittel.

8. Peptid nach einem der Ansprüche 1 bis 5 oder eine Verbindung nach Anspruch 6 zur Verwendung in der Medizin.

9. Verwendung eines Peptids nach einem der Ansprüche 1 bis 5 oder eine Verbindung nach Anspruch 6 für die Herstellung eines Mittels zur Behandlung oder Prophylaxe von Krankheiten, die durch eine verminderte neutrophile Funktion gekennzeichnet sind.

10. Verwendung nach Anspruch 9, wobei das Individuum entweder am erworbenen Immunschwächesyndrom oder an Krebs leidet.

11. Verwendung eines Peptides nach Anspruch 3 für die Herstellung eines Mittels zur Behandlung eines Individuums, das an einem Entzündungssyndrom leidet.

12. Verwendung nach Anspruch 11, wobei das Entzündungssyndrom, an dem das Individuum leidet, rheumatoide Arthritis oder die akute respiratorische Insuffizienz ist.

## Revendications

1. Peptide ayant une séquence d'acides aminés correspondant aux acides aminés 54 à 94 de la Figure 1, ou un partie de celle-ci, le peptide étant capable de sensibiliser des leucocytes neutrophiles en vue d'une production de superoxyde et d'une activité respiratoire rehaussée après un traitement par la N-formyl-L-méthionyl-L-leucyl-L-phénylalanine.

2. Peptide selon la revendication 1, dans lequel le peptide a une séquence d'acides aminés correspondant aux acides aminés 54 à 94 de la Figure 1.

3. Peptide selon la revendication 1, dans lequel le peptide a une séquence d'acides aminés correspondant aux acides aminés 54 à 68 de la Figure 1.

4. Peptide selon la revendication 1, dans lequel le peptide a une séquence d'acides aminés correspondant aux acides aminés 73 à 94 de la Figure 1.

5. Peptide selon la revendication 1, dans lequel le peptide a une séquence d'acides aminés correspondant aux acides aminés 70 à 80 de la Figure 1.

6. Composé ayant une structure tridimensionnelle qui correspond à la structure tridimensionnelle du peptide tel que défini à l'une quelconque des revendications 1 à 5, lequel composé est capable de sensibiliser des leucocytes neutrophiles en vue d'une activité respiratoire après un traitement par la N-formyl-L-méthionyl-L-leucyl-L-phénylalanine.

7. Formulation pharmaceutique comprenant un peptide tel que défini à l'une quelconque des revendications 1 à 5 et n'importe quel support ou diluant pharmaceutiquement acceptable.

8. Peptide selon l'une quelconque des revendications 1 à 5 ou composé selon la revendication 6, pour utilisation en médecine.

9. Utilisation d'un peptide tel que défini à l'une quelconque des revendications 1 à 5 ou d'un composé tel que défini à la revendication 6 dans la préparation d'un agent pour le traitement ou la prophylaxie de maladies caractérisées par une fonction des leucocytes neutrophiles diminuée.

10. Utilisation selon la revendication 9, dans laquelle le sujet est atteint soit du syndrome immunodéficitaire acquis, soit d'un cancer.

11. Utilisation d'un peptide tel que défini à la revendication 3 dans la préparation d'un agent pour le traitement d'un sujet atteint d'un syndrome inflammatoire.

12. Utilisation selon la revendication 11, dans laquelle le syndrome inflammatoire dont le sujet est atteint est la polyarthrite rhumatoïde ou le syndrome de détresse respiratoire aiguë de l'adulte.
